# EUROPEAN PATENT SPECIFICATION

(11) **EP 4 684 395 B1**
(45) Date of publication and mention of the grant of the patent: **07.10.2026**
(21) Application number: 24739307.7
(22) Date of filing: 05.06.2024
(51) Int. Cl.: G16B 20/00, G16B 40/20, G16H 50/20, G16H 50/30

(54) **METHODS AND SYSTEMS FOR ESTIMATING THE AGE OF A HUMAN SUBJECT FROM THE HUMAN SUBJECT BIOLOGICAL SAMPLE**
VERFAHREN UND SYSTEME ZUR SCHÄTZUNG DES ALTERS EINER MENSCHLICHEN PERSON AUS EINER HER-BIOLOGISCHEN PROBE
PROCÉDÉS ET SYSTÈMES D'ESTIMATION DE L'ÂGE D'UN SUJET HUMAIN D'APRÈS SON ÉCHANTILLON BIOLOGIQUE

(43) Date of publication of application: 28.01.2026
(73) Proprietor: GENETON s.r.o., 841 04 Bratislava (SK)
(72) Inventor: KRAMPL, Werner, 831 06 Bratislava (SK); BUDIS, Jaroslav, 900 31 Stupava (SK); SZEMES, Tomás, 841 03 Bratislava (SK); BUCHALOVÁ, Monika, 040 13 Kosice (SK); BOKOROVÁ, Silvia, 851 01 Bratislava (SK); HANZLÍKOVÁ, Zuzana, 966 03 Sklené Teplice (SK); PÖS, Ondrej, 930 41 Hviezdoslavov (SK); STYK, Jakub, 831 06 Bratislava (SK); SEDLÁCKOVÁ, Tatiana, 922 03 Vrbové (SK); RADVÁNSZKY, Ján, 930 41 Hviezdoslavov (SK)
(74) Representative: inventa Patentova a znamkova kancelaria s.r.o.
(86) International application number: PCT/SK2024/050006
(87) International publication number: WO 2025/254600

(56) References cited:
- US-A1- 2020 075 127
- US-A1- 2022 344 003
- US-A1- 2024 110 233

## Description

### Field of the invention

The present invention relates to the field of DNA sequencing, genomics and bioinformatics, specifically to methods and systems for predicting the age of a human subject her biological sample using sequencing data. More particularly, the present invention involves the analysis of genetic markers to estimate chronological age with high accuracy and relates to a computer-implemented method for estimating the age of a human subject from the human subject biological sample, a trained model for estimating the age of human subject from the human subject biological sample, a use of the trained model and a computer system comprising computing means configured to perform said method. It further relates to fields of molecular biology and biotechnology.

### Background of the invention

### Genome analysis

The human genome is physically stored in a double helix DNA molecule, consisting of two strands, each carrying a sequence of nucleotides (adenine [A], thymine [T], guanine [G], and cytosine [C]) known as bases. A complete human genome comprises approximately 3.2 billion DNA bases. The reference genome, an artificial construct created by scientists, represents the most common sequence of bases in human DNA. Individual genomes differ from the reference genome by about 0.5% of the bases due to genetic variations. These variations make each genotype unique and can significantly impact human health.

Genome analysis begins with the collection of a biological sample (e.g., blood, saliva) from which the DNA is extracted and prepared for a process called sequencing. DNA sequencing is a biochemical process used to determine the precise order of nucleotide bases within a DNA molecule. Using massive parallel sequencing technology (Mayer et al., 1998), the DNA molecule is fragmented and placed on a sequencing platform. The fragments are read in parallel, creating digital sequences of DNA bases known as reads. To detect genomic variants in the human genome, over a billion reads may be required (Kim et al., 2015). These reads are randomly ordered with unknown direction and DNA strand of origin.

When sequencing the genome of an organism with a known reference genome, such as humans, the reads can be organized through a process called mapping. The goal of mapping is to reconstruct the original genomic sequence. Each read is aligned to its most probable region of origin on the reference genome. An aligned and mapped read is often referred to as an alignment. A set of aligned reads constitutes a digital copy of the DNA contained within the biological sample. These aligned reads reveal differences between the sequenced genome and the reference genome, known as genomic variants. The complete set or specific selection of these genomic variants is unique to each individual, making the genome the ultimate personal identifier. Although most of these variants have no apparent effect, genome-wide association studies have linked some variants to diseases, physical appearance, and even behaviour.

The human genome is organized into 22 pairs of homologous chromosomes and one pair of sex chromosomes. Each pair consists of one chromosome inherited from the mother and one from the father.

### Age Prediction from Biological Samples

Age prediction from biological samples leverages specific genetic and epigenetic markers that correlate with chronological age. Epigenetic modifications, particularly DNA methylation, have been identified as relatively reliable biomarkers for age estimation. DNA methylation involves the addition of methyl groups to cytosine bases, primarily at CpG sites, and can influence gene expression without altering the underlying DNA sequence. These methylation patterns change predictably with age and can be quantified using sequencing data.

The sequencing process begins with the collection of a biological sample, such as blood or saliva, from which DNA is extracted. Following extraction, the DNA undergoes bisulfite conversion to differentiate between methylated and unmethylated cytosines. The converted DNA is then subjected to sequencing, resulting in data that reflects the methylation status of numerous CpG sites across the genome.

Advanced bioinformatics tools analyse the sequencing data to identify methylation patterns associated with aging. Machine learning algorithms are often employed to construct predictive models that estimate an individual's age based on the methylation data. These models are trained on large datasets that correlate known ages with specific methylation signatures.

Other techniques based on DNA sequencing include telomere length analysis and proteomic analysis.

Telomeres are repetitive nucleotide sequences at the ends of chromosomes that protect them from degradation. Telomere length shortens with each cell division and is considered a marker of cellular aging. By measuring the average length of telomeres in a biological sample, such as blood cells, researchers can estimate the biological age of an individual. Techniques such as quantitative PCR (qPCR) and terminal restriction fragment (TRF) analysis are commonly used for telomere length measurement.

Proteins in the body undergo various changes as a person ages. Proteomic analysis involves studying the protein composition of biological samples to identify age-related changes in protein expression, modification, and degradation. Mass spectrometry is a powerful tool used in proteomics to detect and quantify proteins, providing insights into the biological processes associated with aging.

Beside techniques based on DNA sequencing, other techniques have been developed as well.

The immune system undergoes significant changes with age, a process known as immunosenescence. By analysing markers of immune function, such as the composition and activity of different immune cell types, researchers can estimate biological age. Flow cytometry is a common technique used to assess immune cell populations and their functional state.

Imaging techniques, such as magnetic resonance imaging (MRI) and computed tomography (CT) scans, can provide information on age-related changes in tissues and organs. For example, brain imaging can reveal structural changes associated with aging, while bone density scans can assess age-related bone loss. These imaging biomarkers can be correlated with chronological age to develop predictive models.

### Description of Prior Art

Cassina and Clementi (2017) discuss various DNA-based methods for age estimation in their chapter "DNA-Based Methods for Age Estimation" from the book "P5 Medicine and Justice" edited by S.D. Ferrara. The primary DNA-based methods explored include the analysis of telomere repeats, mitochondrial DNA variants, sjTREC rearrangements in T-cells, and the methylation status of the human genome.

Telomeres are repetitive DNA sequences located at the ends of chromosomes that shorten with each cell division. The measurement of telomere length has been explored as a potential method for age estimation. Various techniques such as Southern blotting, quantitative PCR, and flow-fluorescence in situ hybridization (Flow-FISH) have been employed. Although an inverse correlation between telomere length and age has been observed, the accuracy of this method is limited due to significant variability influenced by genetic and environmental factors. Consequently, the high standard error and variability among individuals and tissues make telomere length measurement insufficiently reliable for forensic age estimation.

Mitochondrial DNA (mtDNA) accumulates mutations at a higher rate than nuclear DNA, making it another candidate for age estimation. Studies have shown age-dependent increases in specific mtDNA deletions and duplications in various tissues. The most commonly studied deletion is a 4977 bp deletion, which correlates with age in tissues such as muscle and brain. However, the broad confidence intervals and variability among tissues limit the precision of age estimation using mtDNA mutations. This method is generally more effective for distinguishing between young and old individuals rather than providing precise age estimates.

Signal joint T-cell receptor excision circles (sjTRECs) are byproducts of T-cell receptor rearrangements that occur during T-cell maturation in the thymus. The quantity of sjTRECs declines with age due to thymic involution. Quantifying sjTRECs in peripheral blood samples has shown promise for age estimation, with several studies demonstrating a correlation between sjTREC levels and chronological age. The method's accuracy varies, with standard errors reported between 8 and 10 years. While sjTREC analysis is promising, further research is needed to understand the genetic and environmental factors influencing sjTREC levels and to confirm the method's validity for forensic applications.

DNA methylation involves the addition of methyl groups to cytosine bases in CpG dinucleotides and is associated with gene regulation. Age-related changes in DNA methylation patterns, particularly at specific CpG sites, have been identified as reliable biomarkers for age estimation. Studies have developed predictive models using methylation profiles, achieving average accuracies with root mean square error ranging from 3.9 to 6.85 years. The most promising results have been obtained from the analysis of blood samples and bloodstains. However, further studies are required to evaluate the accuracy across different tissues and conditions, especially for long-term stored samples and individuals of diverse ethnic backgrounds.

Studies have utilized statistical and machine learning methods to enhance the accuracy of age prediction models. For example, Hannum et al. (2013) analysed the methylome profiles of a cohort of 482 individuals and evaluated the methylation status of 485,577 CpG loci in blood samples. They developed a predictive model of aging that included both methylomic and clinical parameters such as gender and body mass index. The model demonstrated high accuracy, with a mean absolute error of 3.9 years. They identified 71 methylation markers that were highly predictive of age and most of them were located within or near genes associated with aging-related conditions such as Alzheimer's disease, cancer, tissue degradation, DNA damage, and oxidative stress. The model was validated in an independent cohort of 174 individuals, with predictions showing an error of 4.9 years.

Weidner et al. (2014) combined DNA methylation profiles from 575 blood samples across four studies, covering individuals aged 0 to 78 years. They used the same platform to analyse 27,578 CpG sites and built a multivariate linear model based on 102 age-related CpG sites selected by Pearson correlation. This model correlated with chronological age with a mean absolute deviation of 3.34 years and was validated on three other datasets with deviations ranging from 4.02 to 5.79 years. Applying this model to Hannum et al.'s dataset required adjustments due to differences in the methylation analysis platform, resulting in a mean absolute deviation of 4.12 years.

Zbieć-Piekarska et al. (2015) focused on the ELOVL2 gene, analysing 7 CpG sites in a cohort of 303 individuals. Their final linear regression model, which included 2 CpG sites, predicted age with an error of 6.85 years and a mean absolute deviation of 5.03 years. Validation on an additional set of 124 samples showed a slightly higher deviation of 5.75 years. They also observed that the methylation status of ELOVL2 did not change significantly in bloodstains after four weeks of storage at room temperature and remained relatively stable even after 15 years of storage on tissue paper.

The patent US 10,886,008 B2, titled "Methods and Systems for Determining the Biological Age of Samples," addresses innovative approaches to predicting biological age through machine learning and other computational techniques applied to various biological samples. This document outlines several methodologies and systems that enhance the accuracy and applicability of biological age prediction, particularly focusing on machine learning models that utilize diverse types of biological data.

The patent describes systems and methods for determining the biological age of a sample by leveraging machine learning algorithms. These methods involve several steps, including the collection and preparation of biological samples, pre-processing of sample data, application of machine learning techniques, and prediction of biological age.

The methods begin with the selection of biological samples, which can include cell cultures, primary cells, tissue cross-sections, and other types of samples such as blood, muscle, liver, or skin. These samples are prepared through processes such as staining, imaging, and other biochemical assays to highlight specific phenotypic features.

Data from the prepared samples are generated using various assays, including imaging, sequencing, proteomics, and metabolomics. This raw data is then pre-processed to prepare it for machine learning applications. Pre-processing steps may include normalization, signal noise reduction, and feature extraction to enhance the quality and usability of the data.

The core of the invention according to US 10,886,008 B2 lies in the application of advanced machine learning techniques to analyse the pre-processed sample data. Various machine learning models, including deep learning, convolutional neural networks (CNN), and classical machine learning algorithms like support vector machines (SVM), random forests, and gradient boosting, are employed to learn from the data and make accurate predictions about the biological age of the samples. These models are trained using labeled datasets that include chronological age, age-related diseases, and other relevant clinical metadata.

The machine learning-driven predictors analyse the sample data to generate a biological age prediction. These predictions can be used to assess the aging process, identify age-related biomarkers, and evaluate the effects of anti-aging agents.

The patent US20240110233A1, titled "PCR-Based Epigenetic Age Prediction," describes a method and system for predicting the biological age of an individual based on DNA methylation patterns. This method involves extracting DNA from a biological sample and analysing specific CpG sites known to be associated with aging. The DNA methylation levels at these sites are measured using polymerase chain reaction (PCR) techniques, and the resulting data is processed to predict the epigenetic age of the individual.

The method of US20240110233A1 leverages targeted analysis of age-associated CpG sites, which have been identified through comprehensive genomic studies. By focusing on these specific sites, the method aims to provide a reliable and accurate estimation of biological age.

US2022344003A1 discloses a method to estimate a person's biological age by analysing the genetic material from their microbiome (the community of microorganisms living in their body) and their own cells

It is therefore the aim of the present invention to provide a computer-implemented method and system for estimating the age of a human subject from the human subject biological sample which would involve less steps requiring laboratory preparation, require less laboratory consumables and laboratory equipment and supplies as well as to achieve higher accuracy of the estimate.

### Summary of the invention

The present invention is defined by the appended claims.

For the purpose of this disclosure, the invention will be further described using terms listed below in the "Definitions" below. Other technical and scientific terms used herein have the same meaning as commonly understood by the persons skilled in the art of medicine, molecular genetics, molecular biology, bioinformatics and machine learning.

### Definitions

- Genome - The complete set of DNA sequences within an organism.
- Variant/variation - The terms variant or variation herein refers to a difference between a genome and the reference genome.
- Reference genome - Representative example of a species genome upon which sequencing reads are mapped.
- Single Nucleotide Variation (SNV) - variation at a single position in a DNA sequence
- DNA Insertion and deletion (abb. indel) - types of variations where nucleotides are added (insertion) or removed (deletion) from a DNA sequence
- DNA substitution - type of genetic mutation where one or several nucleotides are replaced by another nucleotide or nucleotides
- Copy number variation - Type of structural variation in which segments of genome is repeated and number of repetitions vary from reference genome. In cases, when the CNV represents a duplication (to one or more copies) of the genomic segment, affected individual would carry additional, third copy (or more) of each extracted gene. In case of a deletion, individual would have only a single copy of a gene in the set.
- DNA sequencing - Techniques allowing precise determination of order of nucleic base pairs in organism
- (Sequencing) Reads - A read is an inferred sequence of base pairs (or base pair probabilities) corresponding to all or part of a single DNA fragment. In other words, they are small continuous parts of an individual's DNA. The read should be long enough to serve as a sequence tag, so it can be unambiguously mapped or assigned to a precise location to a reference genome - at least 30-35bp.
- Mapping - Alignment of the sequence information from NGS (i.e. DNA fragment the genomic position of which is unknown) with a matching sequence in reference human genome. This can be done several ways. Reads that does not map uniquely (map to several positions) are usually excluded from the analysis. The alignment is usually done by computer algorithms well known to the persons skilled in the art of molecular biology and bioinformatics.
- SAM/BAM file - File that contains aligned sequencing reads in a text format (SAM) or a compressed binary format (BAM). For every read it contains its mapped position on the reference genome (if the mapping for that read was successful), the mapping quality, sequencing quality (if provided), the location of the paired read (in case of pair-end sequencing), and various other information. It is a standard for storing aligned reads. Each SAM/BAM file is dependent on the reference genome used - this information is stored in the header of the SAM/BAM file.
- FASTQ file - File containing all reads from a sequencer, together with its sequencing quality. This is the standard file format to store this data and it is usually compressed to save disk space. All the modern mapping software accept this format as input.
- Variant calling - process of identifying variations
- VCF file - File that contains variants of an individual in a concise format. The VCF format is known to people skilled in using of the bioinformatic tools as the standard format to store variants of an individual

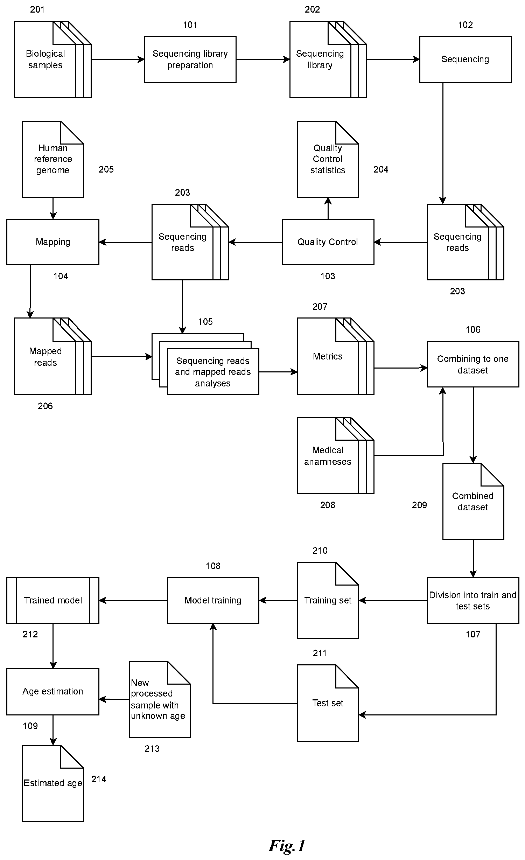

According to a first aspect of the present invention, a computer-implemented method for estimating the age of a human subject from the human subject biological sample is proposed. A schematic overview of the method can be seen in Figure 1.

The process begins with the collection and processing of biological samples, such as blood plasma, saliva, and urine, from a multitude of individuals. Each sample undergoes biochemical preparation for sequencing, involving several steps. Additionally, a medical anamneses of the multitude of individuals is collected, comprising, but not limited to cancer status, acute disease status, organ transplantation status and blood transfusion status. Based on the characteristics of the individuals population, the medical anamneses may include additional data, such as height, weight, sex, BMI, past medical history, current medical status, pregnancy status etc.

Subsequently, DNA is extracted from the individual's biological samples using biochemical and physical techniques known to the skilled person, depending on the sample type. The DNA extracted from the individual's biological samples is then processed into a suitable form for sequencing, typically, but not exclusively, into a sequencing library. The DNA extracted from the individual's biological samples is then sequenced.

Once sequenced, the individual's genome is digitized into sequencing reads, usually stored in a FASTQ file. These reads are checked for quality and, if needed, reads with low quality are removed. Sequencing reads are subsequently mapped to a human reference genome, resulting in a mapped sequencing reads file, usually SAM/BAM file. Mapped reads, together with sequencing reads are further processed by suitable methods known to a skilled person, including, but not limited to, variant calling (both short variants and structural variants), telomere length analysis, microbiome analysis etc. Data created by these processes are subsequently analysed to generate a plurality of metrics comprising, but not limited to, metrics from all of the following categories: short variations (shorter than 50bp), structural variations (equal or larger than 50bp), and telomere length. Preferably, the plurality of metrics comprise metrics from additional categories, such as detected microbiome and mapping statistics. For example, these metrics may include number of DNA sequence substitution, insertion, deletion from short variations and structural variations categories, Escherichia Coli detection from Microbiome detection category, number of mutations from short variations and structural variations categories etc. Medical anamneses collected at the beginning of the process are combined with aforementioned metrics.

Combined metrics and medical anamneses are divided into a training and a test sets for statistical prediction or machine learning purposes, which may or may not include feature selection. A statistical or machine learning prediction model is trained on the training set to classify samples according to age, and its accuracy is validated on the test set. A new, processed biological sample of a human subject is then analysed using this trained and validated model, which results in age estimation for the human subject.

According to another aspect of the invention, a computer system, configured to perform the aforementioned method, is proposed. These method steps can be implemented as modules and submodules within a computer system, which may include computing devices, servers, and communication means (e.g., LAN, internet) for data exchange with other systems and databases. The computing devices and servers preferably have a CPU, GPU, RAM, non-volatile storage (e.g., hard disk), network interfaces, and peripheral devices such as a keyboard and display. Software programs and data are loaded into RAM for processing by the CPU or GPU, generating results for display, output, transfer, or storage.

The modules and submodules may be preferably implemented as computer programs or procedures in common programming languages and executed by the CPU or GPU as object or bytecode. They may also be implemented in hardware, such as integrated circuits or ROM components, enabling each device and server to function as a specialized computer. These programs may be stored on various memory media like HDD, SSD, flash drives, RAM, ROM, etc.

The computer system designed to process age detection samples may include modules for sequencing read processing, variant calling, age-related marker analysis, model training and testing, and new sample classification. Additionally, a computer program product with computer-readable instructions can be loaded and executed to perform these operations. Such computer program product represents another aspect of the present invention.

Preferably, the computer system may either be a single system handling all computations or a server distributing tasks across several computing nodes. Each node preferably performs specific computations and sends the results back to the server.

The present method finds applications in several key areas, including forensic science, clinical research, regenerative medicine, public health, sports science, and the cosmetics industry. **In** forensic science, age prediction plays a crucial role in criminal investigations and legal proceedings. When unidentified human remains are discovered, forensic experts can use age presented method to narrow down potential identities and assist in constructing biological profiles. This information is invaluable in missing persons cases, disaster victim identification, and historical or archaeological investigations. By analysing DNA of biological sample, forensic scientists can provide reliable age estimates that support the identification process.

**In** the cosmetics and wellness industry, the present method might be used to develop and validate anti-aging products. By measuring biological age markers before and after the application of skincare or nutritional products, companies can scientifically demonstrate the efficacy of their products in slowing down or reversing the signs of aging. This scientific validation enhances consumer trust and product credibility.

**In** clinical research, the present age estimation method may be used to study age-related diseases and conditions. Understanding the biological age of individuals can help researchers identify early biomarkers of diseases such as Alzheimer's, cardiovascular diseases, and cancer. By comparing the biological age to the chronological age, researchers can gain insights into the aging process, assess the effectiveness of anti-aging interventions, and develop personalized treatment plans. For example, individuals with a biological age significantly higher than their chronological age may be at greater risk for age-related diseases, prompting more proactive monitoring and preventative measures.

Another important application is in the field of regenerative medicine and stem cell research. Age prediction can be used to evaluate the age of stem cells or tissues used in therapeutic applications. This ensures that younger, more viable cells are selected for treatments, improving the success rates of regenerative therapies. Additionally, age prediction can help in the assessment of donor tissue suitability in organ transplantation, ensuring compatibility and longevity of transplanted organs.

**In** population studies and public health research, presented method can be used to understand demographic trends and the impact of environmental factors on aging. Large-scale epidemiological studies often require accurate age data to analyse health outcomes and the effectiveness of public health interventions. Biological age estimation can provide more precise data than self-reported ages.

Furthermore, the present method may be utilized in sports science and anti-doping efforts. Understanding an athlete's biological age can contribute to the assessment of their physiological state and potential for performance enhancement.

The method according to the present invention, which encompasses using metrics describing short variations, structural variations, telomere length, and medical anamnesis in estimating the age from biological sample has several advantages over traditional methods. Firstly, the present method does involve fewer steps and is generally less complex. Analysing short variations, structural variations and telomere length requires less laboratory preparation steps in comparison to methods based on methylation or RNA sequencing, which require additional steps to create the sequencing library. RNA sequencing requires its reverse transcription from RNA to DNA and methylation methods require bisulfide treatment step.

Age estimation according to the present method is based on the combined metrics for each type of variation from aforementioned categories, as these generally increase in the individual's genome as can be seen in table 1. Medical anamneses are used to further increase the accuracy of the estimation, as these further describe the source of variations. Metrics describing detected microbiome and mapping may be further used to increase accuracy of the estimation.

Moreover, the costs, associated with estimating age of a subject are significantly lower when using present method when comparing the costs, associated with traditional methods. DNA sequencing, especially for detecting short variations, structural variations and telomere length, is relatively inexpensive due to lower demands on laboratory consumables and supplies, compared to the high expenses associated with comprehensive epigenetic profiling or RNA sequencing, which require more specialized equipment, reagents, and computational resources for data analysis.

**Table 1**

| **age** | **Average number of indels** | **Average minimal telomere length** | **Average number of CNVs** |
|---|---|---|---|
| 18 | 176977731 | 45.77777778 | 1.888888889 |
| 19 | 176747143.1 | 52.58823529 | 1.176470588 |
| 20 | 174504035.6 | 65.57142857 | 1.035714286 |
| 21 | 174619310.4 | 65.03125 | 1.25 |
| 22 | 175462735.4 | 66.66666667 | 2.977777778 |
| 23 | 172436620.5 | 64.58974359 | 1.333333333 |
| 24 | 173330239.7 | 66.92682927 | 2.268292683 |
| 25 | 176081754.3 | 63.2295082 | 1.327868852 |
| 26 | 177221567.7 | 62.41176471 | 1.823529412 |
| 27 | 179651945.6 | 63.86363636 | 1.318181818 |
| 28 | 174219118.3 | 65.4137931 | 1.551724138 |
| 29 | 181434881.8 | 61.23529412 | 4.5 |
| 30 | 176576063.6 | 63.71875 | 1.625 |
| 31 | 175856468.3 | 64.82142857 | 1.392857143 |
| 32 | 176345482.1 | 59.76470588 | 1.617647059 |
| 33 | 180759661 | 61 | 3.823529412 |
| 34 | 183759079.6 | 66.5952381 | 6.452380952 |
| 35 | 177091689.3 | 66.30769231 | 1.884615385 |
| 36 | 177776105.9 | 64.83333333 | 1.8 |
| 38 | 167430220.4 | 57 | 1.9 |

### Overview of figures on the drawings

The method according to the present invention is illustrated the drawing, where:
- Fig. 1: Depicts a schematic diagram of the proposed method from samples and anamneses collection to the estimated age

### Examples embodiments of the invention

The following specific examples of embodiments are provided for illustrative purposes only and should not be understood as limitations on the scope of protection provided by the patent claims.

### Example 1

Peripheral blood samples from totally 231 individuals were collected into 10 mL EDTA tubes together with their medical anamneses data 208. Plasma was separated from the blood through a two-step centrifugation process: first at 2200g for 8 minutes, followed by 16,000g for 8 minutes. DNA was extracted using the QIAamp DNA Blood Mini Kit (Qiagen). The extracted DNA was quantified with the Qubit dsDNA HS Assay Kit (Thermo Fisher Scientific), ensuring a maximum DNA concentration limit of 0.3 ng/µL for samples collected in EDTA tubes.

Preparation 101 DNA sequencing libraries 202 using the TruSeq Nano DNA Library Prep Kit (Illumina). Sequencing 102 was performed on a NextSeq 2000 platform with the NextSeq 1000/2000 P3 (200 cycles) from Illumina, utilizing a paired-end sequencing protocol (2 x 100 cycles), resulting in sequencing reads 203 available for further processing.

All readings from the dataset of colorectal cancer patients and healthy controls are analysed using next-generation sequencing (NGS) technology, namely Illumina's sequencing platform with the Truseq sequencing kit with 100 bp reads, yielding datasets in FASTQ format.

### Example 2

In this example, data from totally 80 sequenced male samples were used together with their medical anamneses data 208. Once sequenced 102, sequencing reads 203 were checked for quality using FastQC tool and resulting data is extracted. Following this step, sequencing reads 203 are mapped 104 to a human reference genome 205, GRCh38 using BWA, resulting in on the BAM file. The quality of the mapped reads 206 is checked with QualiMap and resulting data is extracted.

The mapped reads 206 are further processed by several methods, including variant calling with DeepVariant and Wisecondor, telomere length analysis using TelomereHunter, and microbiome analysis with Kraken. Custom-made scripts are employed for further analysis 105 of resulting data from Short variations, Structural variations, Telomere length, Microbiome detection categories to extrapolate a plurality of metrics 207 such as total number of SNV where C changes to A etc.

The plurality of metrics 207, together with medical anamneses data 208, are processed by Principal Component Analysis which results in 16 features for every sample. This combined dataset 209 is divided 107 into a training set 210 and a test set 211 for machine learning purposes, following a ratio of 0.8 to 0.2. The training set 210, comprising 80% of the combined dataset 209, is used to train 108 a model to classify samples according to age. The remaining 20% of the combined dataset 209 is used as the test set 211 to validate the accuracy of the model. Machine learning model used in this example is the Extreme Gradient Boosting model. The Root Mean Square Error (RMSE) on the test set 210 was calculated as 5.16. This example results in trained model 212 prepared to estimate age of new, unknown biological sample 213 of a human subject.

A biological sample 213 of a male human subject with unknown age was provided and after laboratory preparation, DNA sequencing and bioinformatical processing, it analysed by the trained model and the estimated age 214 was found to be 25 years.

### Example 3

In this example, data from totally 151 sequenced female samples were used together with their medical anamneses data 208. Once sequenced 102, sequencing reads 203 are checked for quality 103 using FastQC tool and resulting data is extracted. Following this step, sequencing reads 203 are mapped 104 to a human reference genome 205, GRCh38 using BWA, resulting in the BAM file. The quality of the mapped reads 206 is checked with QualiMap and resulting data is extracted.

The mapped reads 206 are further processed by several methods, including variant calling with DeepVariant and Wisecondor, telomere length analysis using TelomereHunter, and microbiome analysis with Kraken. Custom-made scripts are employed for further analysis 105 of resulting data from Short variations, Structural variations, Telomere length, Microbiome detection categories to extrapolate a plurality metrics 207 such as total number of SNV where C changes to A etc.

The plurality of metrics 207, together with medical anamneses data 208, are processed by Principal Component Analysis which results in 16 features for every sample. This combined dataset 209 is divided into a training set 210 and test sets 211 for machine learning purposes, following a ratio of 0.8 to 0.2. The training set 210, comprising 80% of the combined dataset 209, is used to train 108 a model to classify samples according to age. The remaining 20% of the combined dataset 209 is used as the test set 211 to validate the accuracy of the model. Machine learning model used in this example is Gradient Boosting Regressor model. The Root Mean Square Error (RMSE) on the test set 210 was calculated as 3.82. This example results in trained model 212 prepared to estimate age of new, unknown biological sample 213 of a human subject.

A biological sample 213 from female human subject with unknown age was provided and after laboratory preparation, DNA sequencing and bioinformatical processing, it analysed by the trained model and the estimated age 214 was found to be 32 years.

### Example 4

A Computer system in this example is a standalone computing server without additional computing nodes. The server comprises of Intel Core i5 - 7300HQ 2.50 GHz, HyperX 8GB DDR4 2666 MHz CL16 FURY series, Western Digital 2TB Ultrastar DC HA210 SATA HDD and a LAN connection to external databases.

Sequencing data enters the computer system together with the human reference genome 205. The quality control 103 of the sequencing reads 203 is performed by the Sequencing Quality Control module incorporating FastQC tool, followed by mapping 104 step of the Mapping module performed by the BWA tool. Various analyses, some of which were described in the invention summary, were performed in the Analysis module.

Subsequent module Feature selection implements Principal Component analysis from Scikit Python3 library. Resulting combined dataset 209 is afterwards divided 107 into a training set 210 and a test set 211 in the Sets creation module and are subsequently processed in the Machine learning module, which incorporates Extreme Gradient Boosting model from XGBoost Python3 library. This example results in trained model 212 prepared to estimate age of a new, unknown biological sample 213 of a human subject.

### Industrial applicability

The method and systems according to the present invention can be used in various fields, including forensic science, clinical research, regenerative medicine, public health, sports science, and the cosmetics industry.

### List of reference numbers

- 101: Preparation of Sequencing library
- 102: Sequencing
- 103: Quality control
- 104: Mapping
- 105: Analysing sequencing reads and mapped reads
- 106: Combining data to a dataset
- 107: Dividing the combined dataset into training set and test set
- 108: Training
- 109: Age estimation
- 201: Biological samples
- 202: Sequencing library
- 203: Sequencing reads
- 204: Quality control statistics
- 205: Human reference genome
- 206: Mapped reads
- 207: Plurality of metrics
- 208: Medical anamnesis data
- 209: Combined dataset
- 210: Training set
- 211: Test set
- 212: Trained model
- 213: Biological sample of a human subject
- 214: Estimated age

## Claims

1. A computer-implemented method for estimating the age of a human subject from the human subject biological sample, **characterized in that** it comprises the following steps:
a. Collecting biological samples (201) from a multitude of individuals;
b. Extracting DNA from the biological samples (201);
c. Sequencing (102) the extracted DNA to generate sequencing reads (203);
d. Mapping (104) the sequencing reads (203) to a human reference genome (205) to produce mapped reads (203);
e. Analysing (105) the sequencing reads (203) and mapped reads (206) to generate a plurality of metrics (207) which characterize the biological samples (201); the plurality of metrics (207) comprise metrics from all of the following categories: short variations shorter 50bp, structural variations equal or larger than 50bp, telomere length;
f. Collecting medical anamnesis (208) data of the said individuals, comprising cancer status, acute disease status, organ transplantation status and blood transfusion status;
g. Combining the plurality of metrics (207) from step e and medical anamnesis (208) data from step f into a combined dataset (209);
h. Dividing (107) the combined dataset (209) into a training set (210) and a test set (211);
i. Training (108) a statistical or a machine learning model on the training set (210) to estimate (109) the age of a human subject;
j. Validating the accuracy of the statistical or the machine learning model using the test set (211);

2. The method according to claim 1, wherein the biological samples (201) is selected from a group comprising of blood, saliva, urine, cerebrospinal fluid, tissue.

3. The method according to any of the previous claims, wherein the medical anamnesis (208) data further comprise data selected from a group comprising height, weight, sex, BMI, past medical history, current medical status, pregnancy status.

4. The method according to any of the previous claims, wherein the age is a chronological age.

5. The method according to any of the claims 1 to 4, wherein the age is a biological age.

6. The method according to any of the preceding claims, wherein the plurality of metrics (207) comprise metrics from the following categories: detected microbiome, mapping statistics.

7. A trained model (212) for estimating the age of human subject from the human subject biological sample (213), trained (108) using the method according to any of the previous claims.

8. A use of the trained model (212) according to claim 7 for estimating (109) an estimated age (214) of a human subject.

9. The use of the trained model (212) according to claim 8, where the estimated age (214) is a chronological or a biological age.

10. A computer system comprising computing means configured to perform the method according to any one of claims 1 to 6, or to use the trained model (212) according to claim 7.

11. A computer program comprising instructions which, if executed by a computer, ensure the implementation of the method according to any one of claims 1 to 6 or the use according to claims 8 to 9, by this computer.

12. A computer data medium comprising program instructions which, when executed by a computer, ensure the implementation of the method according to any one of claims 1 to 6, or the use according to claims 8 to 9, by this computer.

## Patentansprüche

1. Eine computerimplementierte Methode zur Altersschätzung eines menschlichen Subjekts aus der biologischen Probe, **dadurch gekennzeichnet, dass** sie folgende Schritte umfasst:
a. Sammeln biologischer Proben (201) von mehreren Individuen;
b. DNA-Extraktion aus den biologischen Proben (201);
c. Sequenzierung (102) der extrahierten DNA zum Generieren von Sequenzierungs-Reads (203);
d. Mapping (104) der Sequenzierungs-Reads (203) auf das menschliche Referenzgenom (205) zur Bildung der gemappten Reads (203);
e. Analyse (105) der Sequenzierungs-Reads (203) und der gemappten Reads (206) zum Generieren einer Menge der Metriken (207), die durch biologische Proben (201) charakterisiert werden; wobei die Menge der Metriken (207) Metriken aus allen folgenden Kategorien umfasst: kurze Variationen kürzer als 50 bp, strukturelle Variationen gleich oder größer als 50 bp, Telomerlänge;
f. Datenerfassung aus der ärztlichen Anamnese (208) der angeführten Individuen, einschließlich Krebsstatus, Status der akuten Erkrankung, Status der Organtransplantation und Status der Bluttransfusion;
g. Kombinieren von mehrerer Metriken (207) aus dem Schritt e und aus den Daten der ärztlichen Anamnese (208) aus dem Schritt f in einen kombinierten Datenbestand (209);
h. Verteilung (107) des kombinierten Datenbestands (209) in einen Trainingssatz (210) und einen Testsatz (211);
i. Trainieren (108) des statistischen oder maschinellen Lernmodells auf dem Trainingssatz (210) zur Altersschätzung (109) eines menschlichen Subjekts;
j. Validierung der Genauigkeit des statistischen oder des maschinellen Lernmodells mithilfe des Testsatzes (211).

2. Methode nach Anspruch 1, wobei die biologischen Proben (201) aus einer Gruppe ausgewählt sind, die Blut, Speichel, Urin, Zerebrospinalflüssigkeit und Gewebe umfasst.

3. Methode nach irgendeinem der vorhergehenden Ansprüche, wo die Daten aus der ärztlichen Anamnese (208) weiter die Daten umfassen, die aus der Gruppe ausgewählt sind, die Größe, Gewicht, Geschlecht, BMI, vorherige Anamnese, den aktuellen Gesundheitszustand und den Schwangerschaftszustand umfasst.

4. Methode nach irgendeinem der vorhergehenden Ansprüche, wo das Alter das chronologische Alter ist.

5. Methode nach irgendeinem der Ansprüche 1 bis 4, wo das Alter das biologische Alter ist.

6. Methode nach irgendeinem der vorhergehenden Ansprüche, wo mehrere Metriken (207) die Metriken aus folgenden Kategorien umfassen: detektiertes Mikrobiom, Mapping-Statistiken.

7. Ein zur Altersschätzung eines menschlichen Subjekts aus der biologischen Probe (213) eines menschlichen Subjekts trainiertes Modell (212), trainiert (108) mithilfe einer Methode nach irgendeinem der vorhergehenden Ansprüche.

8. Verwendung des trainierten Modells (212) nach Anspruch 7 zur Schätzung (109) des zu schätzenden Alters (214) eines menschlichen Subjekts.

9. Verwendung des trainierten Modells (212) nach Anspruch 8, wo das zu schätzende Alter (214) das chronologische oder das biologische Alter ist.

10. Ein Computersystem, das Rechenmittel enthält, die konfiguriert sind, um die Methode nach irgendeinem der Ansprüche 1 bis 6 durchzuführen oder das trainierte Modell (212) nach Anspruch 7 zu verwenden.

11. Ein Computerprogramm, das Anweisungen enthält, die, falls sie von einem Computer ausgeführt werden, die Implementierung der Methode nach irgendeinem der Ansprüche 1 bis 6 oder die Verwendung nach Ansprüchen 8 bis 9 von diesem Computer sicherstellen.

12. Ein Computerdatenmedium, das Programmanweisungen enthält, die, wenn sie von einem Computer ausgeführt werden, die Implementierung der Methode nach irgendeinem der Ansprüche 1 bis 6 oder Verwendung nach Ansprüchen 8 bis 9 von diesem Computer gewährleisten.

## Revendications

1. Procédé mis en œuvre par ordinateur pour estimer l'âge d'un sujet humain à partir d'un échantillon biologique, **caractérisé en ce qu'il** comprend les étapes suivantes:
a. la collecte d'échantillons biologiques (201) auprès d'une pluralité d'individus;
b. l'extraction d'ADN des échantillons biologiques (201) (201);
c. le séquençage (102) de l'ADN extrait pour générer des lectures de séquençage (203);
d. le mappage (104) des lectures de séquençage (203) sur un génome humain de référence (205) pour créer des lectures mappées (203);
e. l'analyse (105) des lectures de séquençage (203) et des lectures mappées (206) pour générer une pluralité de métriques (207) qui caractérisent les échantillons biologiques (201) ; dans lequel la pluralité de métriques (207) comprend des métriques de toutes les catégories suivantes : variations courtes inférieures à 50 pb, variations structurelles égales ou supérieures à 50 pb, longueur des télomères;
f. la collecte de données d'antécédents médicaux (208) desdits individus, comprenant l'état cancéreux, l'état de maladie aiguë, les antécédents de greffe d'organe et les antécédents de transfusion sanguine;
g. la combinaison de la pluralité de métriques (207) de l'étape e et des données d'antécédents médicaux (208) de l'étape f en un ensemble de données combiné (209);
h. la division (107) de l'ensemble de données combiné (209) en un ensemble d'apprentissage (210) et un ensemble de test (211);
i. l'entraînement (108) d'un modèle statistique ou d'apprentissage automatique sur l'ensemble d'apprentissage (210) pour estimer (109) l'âge du sujet humain;
j. la validation de la précision du modèle statistique ou d'apprentissage automatique à l'aide de l'ensemble de test (211).

2. Procédé selon la revendication 1, dans lequel les échantillons biologiques (201) sont choisis dans le groupe comprenant le sang, la salive, l'urine, le liquide céphalo-rachidien, les tissus.

3. Procédé selon l'une quelconque des revendications précédentes, dans lequel les données d'antécédents médicaux (208) comprennent en outre des données choisies dans le groupe comprenant la taille, le poids, le sexe, l'IMC, les antécédents médicaux antérieurs, l'état de santé actuel et l'état de grossesse.

4. Procédé selon l'une quelconque des revendications précédentes, dans lequel l'âge est l'âge chronologique.

5. Procédé selon l'une quelconque des revendications 1 à 4, dans lequel l'âge est l'âge biologique.

6. Procédé selon l'une quelconque des revendications précédentes, dans lequel la pluralité de métriques (207) comprend des métriques issues des catégories suivantes : microbiome détecté, statistiques de mappage.

7. Modèle entraîné (212) pour estimer l'âge d'un sujet humain à partir d'un échantillon biologique (213) du sujet humain, entraîné (108) à l'aide du procédé selon l'une quelconque des revendications précédentes.

8. Utilisation du modèle entraîné (212) selon la revendication 7 pour estimer (109) l'âge estimé (214) d'un sujet humain.

9. Utilisation du modèle entraîné (212) selon la revendication 8, dans laquelle l'âge estimé (214) est l'âge chronologique ou l'âge biologique.

10. Système informatique comprenant des moyens de calcul configurés pour exécuter le procédé selon l'une quelconque des revendications 1 à 6 ou pour utiliser le modèle entraîné (212) selon la revendication 7.

11. Programme d'ordinateur comprenant des instructions qui, lorsqu'elles sont exécutées par un ordinateur, conduisent à la mise en œuvre du procédé selon l'une quelconque des revendications 1 à 6 ou à l'utilisation selon les revendications 8 et 9 par ledit ordinateur.

12. Support de données informatique comprenant des instructions de programme qui, lorsqu'elles sont exécutées par un ordinateur, conduisent à la mise en œuvre du procédé selon l'une quelconque des revendications 1 à 6, ou à l'utilisation selon les revendications 8 et 9 par ledit ordinateur.
